# EUROPEAN PATENT APPLICATION

(11) **EP 2 700 321 A1**
(43) Date of publication of application: **26.02.2014**
(21) Application number: 12306021.2
(22) Date of filing: 24.08.2012
(51) Int. Cl.: A23L 1/10, A23L 1/05, A23L 1/0524, A23L 1/053, A23L 1/054, A23L 1/308, A23L 2/00, A23L 2/52, A61K 31/00

(54) **Aqueous food composition enriched in beta-glucan**

(71) Applicant: Etablissements J. Soufflet, 10400 Nogent Sur Seine (FR)
(72) Inventor: Devaux, Laurence, 10400 Nogent sur Seine (FR); Perrin, Aurore, 10400 Nogent sur Seine (FR)
(74) Representative: Domenego, Bertrand

(57) **Abstract**

The present invention relates to an aqueous food composition, comprising at least one β-glucan polymer and at least one mixture of stabilising agents, wherein the β-glucan polymer is present in an amount from 0.4% to 3% by weight of the composition.

## Description

The present invention relates to aqueous food compositions comprising β-glucan polymers, processes for preparing such compositions and uses of such compositions.

The term "β-glucan polymer", also further called "β-glucan", refers to polysaccharide-type polymers comprising D-glucopyranosyl units linked together by (1→3) and (1→4) p-linkages. β-Glucans occur naturally in many cereal grains such as oats and barley.

β-Glucan is desirable as a food additive, for example, to impart texture ("mouth feel") to foods. β-Glucan also has the advantage of having a neutral flavour.

β-Glucan is also desirable as a therapeutic agent. β-Glucan, particularly from oats and barley grains, has been linked to a number of beneficial effects, for example the reduction of serum cholesterol levels, alongside improvements in HDL/LDL ratios in blood, an effect strongly correlated with improved cardiovascular health in humans (Bell et al., Crit. Rev. Food Sci. Nutr., Vol 39, 2, 1999). β-Glucan has also been shown to provide cardiovascular benefits, by preventing atherosclerosis and coronary heart diseases (Keogh et al., Am. J. Clin. Nutr. 2003, 78: 711-718).

According to the Food and Drug Administration (CFR, Title 21, §101.81), the daily intake of β-glucan to achieve a significant reduction of cholesterol has to be at least 3 g. The European Food Safety Authority (EFSA Journal 2009; 7(9):1254) also acknowledges a health claim for the same daily dose of β-glucan.

It is even preferable, for controlling more efficiently fluctuations of blood glucose and insulin, that the amount of β-glucan reach 5 to 6 g per meal (Tappy et al., Diabetes Care 19, 831-834, 1996).

The use of cereal soluble fibre for foods has so far been limited mainly to solid food products, such as breakfast cereals and to certain bakery products, but the amounts of cereal products to be ingested daily have been so high, that only few persons can be persuaded to follow such diets regularly or for long periods.

There is thus a need for new food compositions comprising high amounts of β-glucan that are suitable for the administration of a daily intake of at least 3 g of β-glucan.

The present invention aims at providing an aqueous food composition enriched in β-glucan, conveniently a drinkable aqueous food composition comprising at least 3 g of β-glucan per serving.

However, it is known that, over a concentration in solution of 0.5-1% by weight, β-glucan tends to form a viscous solution or a gel when dissolved in water (WO 02/02645). For example, nectar-type drinks comprising around 1.3% of β-glucan were prepared (WO 01/26479) but rapidly turned to a jelly-like product after 5 min. So far, β-glucan was thus considered by the skilled person to be unsuitable for the preparation of a drinkable aqueous composition comprising a high amount of β-glucan, typically around 0.5-1% by weight of the composition.

The present invention also aims at providing an aqueous food composition enriched in β-glucan, conveniently a drinkable aqueous food composition comprising at least 3 g of β-glucan per serving, which is stable and drinkable for at least one month when kept at usual storage conditions.

The present invention arises from the unexpected finding by the inventors that the addition of a mixture of stabilising agents to a solution of β-glucan enables the stabilisation of said solution, even though the β-glucan is present in an amount of 0.4% or more by weight, at which said solution would usually be unstable in the absence of a mixture of stabilising agents.

The present invention therefore relates to an aqueous food composition, comprising at least one β-glucan polymer and at least one mixture of stabilising agents, wherein the β-glucan polymer is present in an amount from 0.4% to 3% by weight of the composition.

The present invention also relates to a method for preparing a composition according to the present invention, comprising a step of forming an aqueous solution comprising at least one β-glucan polymer and at least a mixture of stabilising agents.

The present invention also relates to a beverage unit comprising an amount of composition according to the present invention such that said beverage unit comprises an amount of β-glucan polymer of at least 3 g.

The present invention also relates to a composition according to the present invention for use for the prevention of a cardiovascular disease or metabolic syndrome in a subject.

The present invention also relates to a composition according to the present invention for use for maintaining a normal blood cholesterol concentration in a subject.

The present invention also relates to a composition according to the present invention for use for decreasing glycemic index of a food or a meal ingested by a subject.

### Detailed description of the invention

### Compositions

In the composition according to the present invention, the amount in β-glucan polymer in the composition is preferably from 0.5 to 2.5%, from 0.6 to 2.0%, from 0.6 to 1.9%, from 0.7 to 1.8%, from 0.7 to 1.7%, from 0.8 to 1.6%, from 0.9% to 1.5%.

Advantageously, the amount in β-glucan polymer in the composition is from 1.0% to 1.5%.

More advantageously, the amount in β-glucan polymer in the composition is typically 1.0%, 1.1%, 1.2%, 1.3%, 1.4% or 1.5%.

Within the framework of the present application, the term "aqueous food composition" relates to cold or hot drinks, optionally slightly jellified, and drinkable in a glass, a cup, a bottle, a can, a doypack, a metal box, a Tetra brick packaging, with a spoon or with a straw.

The compositions according to the invention are highly concentrated in β-glucan and thus suitable for a daily administration of at least 3 g of β-glucan polymer per serving, for example in the framework of a cholesterol-reducing diet.

The term "serving" refers to a portion of the composition that can be ingested during the same day, particularly during a 12 h period. A serving typically represents from 200 mL to 500 mL, preferably from 200 mL to 330 mL. A serving is either taken with a meal or outside meals.

The presence of a mixture of stabilising agents in the composition enhances and extends the stability, the drinkability and the attractive aspect of said composition for a storage period of at least one month.

When kept in usual storage conditions, i.e. at a temperature from 4°C to 10°C, the composition is stable and remains drinkable for at least one month, and preferably for at least 3 months.

The term "stable" means that the composition keeps the aspect obtained just after its preparation, without any aggregation, sedimentation or phase separation, that would affect the aspect of the composition and its drinkability.

The term "aggregation" refers to the formation of particles aggregates, notably gel aggregates, visible to the naked eye, which are unpleasant for the visual aspect of the composition and its drinkability.

The term "sedimentation" refers to the gravity deposition of particles or aggregates at the bottom of the composition.

The term "phase separation" refers to the formation of two or more different phases in the composition, generally with different densities.

The term "β-glucan polymer", also further called "β-glucan", refers to polysaccharide-type polymers comprising D-glucopyranosyl units linked together by (1→3) and (1→4) p-linkages. β-Glucans occur naturally in many cereal grains such as oats and barley.

The average molecular weight of the β-glucan polymer of the composition is typically from 70 kDa to 2 000 kDa.

Preferably, the average molecular weight of the β-glucan polymer is from 100 kDa to 1 500 kDa, from 150 kDa to 1 000 kDa, advantageously from 180 kDa to 500 kDa, from 200 kDa to 350 kDa, still advantageously from 210 kDa to 280 kDa.

The average molecular weight of the β-glucan polymer is generally measured using the method of High Performance Steric Exclusion Chromatography (HPSEC), with a system comprising a refractive index detector and a viscometer coupled with a multi-angles light scattering detector (Viscotek T270).

The β-glucan polymer may be obtained from cereals of the *Pooideae* subfamily. The *Pooideae* subfamily belongs to the Poaceae family and includes in particular the *Triticeae* tribe, the *Aveneae* tribe and the *Brachypodiae* tribe.

Cereals of the *Triticeae* tribe include species from the *Aegilops, Agropyron, Amblyopyrum, Australopyrum, Cockaynea, Crithopsis, Elymus, Elytrigia, Eremium, Eremopyrum, Festucopsis, Haynaldia, Henrardia, Heteranthelium, Hordelymus, Hordeum, Hystrix, Kengyilia, Leymus, Lophopyrum, Malacurus, Pascopyrum, Peridictyon, Psathyrostachys, Pseudoroegneria, Secale, Sitanion, Stenostachys, Taeniatherum, Thinopyrum, Triticosecale* and *Triticum* genus. Cereals of the *Triticeae* tribe used in the context of the invention include in particular species of the *Triticum* genus (including wheat), of the *Hordeum* genus (including barley) and of the *Triticosecale* genus (including triticale). Preferably, the cereal of the *Triticeae* tribe used in the context of the invention is selected from the group consisting of barley and triticale, and is advantageously barley.

Cereals of the *Aveneae* tribe include species from the *Agrostis, Alopecurus, Ammophila, Amphibromus, Anthoxanthum, Apera, Arrhenatherum, Avena, Beckmannia, Calamagrostis, Deschampsia, Dichelachne, Dissanthelium, Gastridium, Gaudinia, Helictotrichon, Holcus, Koeleria, Limnodea, Lophochloa, Mibora, Micropyropsis, Phalaris, Phleum, Rostraria, Sonderina, Sphenopholis, Trisetum, Vahlodea* and Veintenata genus. Cereals of the *Aveneae* tribe used in the context of the invention include in particular species of the *Avena* genus (including oats). Preferably, the cereal of the *Aveneae* tribe used in the context of the invention is oats.

Cereals of the *Brachypodie* tribe include species from the *Brachypodium* genus.

Preferably, the cereal used in the context of the invention is selected from the group consisting of oats, barley, triticale and brachypodium. More preferably, the cereal used in the context of the invention is selected from the group consisting of oats and barley.

The β-glucan polymer is typically obtained from rolled cereals or cereal flour, oats bran, rolled oats, whole oats flour, whole grain barley, dry milled barley, aqueous barley or oats extract, or purified oats or barley β-glucans.

Purified oat or barley β-glucans are commercially available.

Glucagel™, commercialised by DKSH, is obtained from barley and is a convenient source of β-glucan polymer. The average molecular weight of β-glucan polymers in Glucagel™ is 250 kDa.

Barliv™, commercialised by Cargill, is another commercially available source of β-glucan.

Alternatively, the β-glucan polymer is directly obtained from whole grain cereals, rolled cereals or cereal flour, according to a process of aqueous extraction.

The term "stabilising agents", also called "stabilisers", refers to substances, mineral or organic, natural or artificial, which tend to inhibit the reaction between others chemicals. In the food industry, stabilising agents help to preserve the structure of food products. Stabilising agents are generally used in aqueous solutions to control the texture and sensory qualities of said solutions as well as extending their shelf life, by preventing aggregation, sedimentation or phase separation phenomenon.

The term "mixture of stabilising agents" refers to a mixture of different stabilising agents, preferably a mixture of at least two stabilising agents of different type, preferably having different properties.

Preferably, the mixture of stabilising agents of the composition comprises at least a hydrocolloid.

The term "hydrocolloid" refers to a colloid system wherein the colloid particles are hydrophilic polymers dispersed in water. A hydrocolloid has colloid particles spread throughout water and can take place in different states, for example a gel or a jellified solid. In the food industry, hydrocolloids are typically used to influence the texture or viscosity of aqueous preparations, generally by converting liquid preparations into gel or even solid preparations.

The hydrocolloid is preferably selected from the group consisting of arabic gum, xanthan gum, pectin, carrageenans, guar gum, agar, locust bean gum, carboxymethycellulose, microcrystalline cellulose and modified starch.

More preferably, the mixture of stabilising agents of the composition comprises at least a hydrocolloid selected from the group consisting of arabic gum, xanthan gum and pectin.

Preferably, the mixture of stabilising agents is a mixture of xanthan gum and arabic gum, a mixture of xanthan gum and a pectin or a mixture of arabic gum and a pectin.

Most preferably, the mixture of stabilising agents is a mixture of xanthan gum, arabic gum and a pectin.

Preferably, the mixture of stabilising agents of the composition comprises at least a hydrocolloid with thickening properties, for example xanthan gum.

Thixogum S™, commercialised by Nexira is a blend of highly purified xanthan gum and arabic gum (1:1) and is suitable to stabilize the composition according to the invention.

Grindsted Pectin RS 461 S1 and Pectin RS 450, commercialised by Danisco, are commercially available purified pectins and are also suitable to stabilize the composition according to the invention, particularly in association with xanthan gum and/or arabic gum.

Surprisingly, the inventors have found that the addition of a mixture of stabilising agents, preferably comprising at least a hydrocolloid with thickening properties, enables the stabilisation of a solution of β-glucan, even in an amount over 0.4%, and prevents that it turned into a non pourable jelly-like composition for a period of time of at least one month. The mixture of stabilising agents also prevents sedimentation of particles, formation of unpleasant aggregates and phase separation during its storage period.

The mixture of stabilising agents is typically present in an amount from 0.05% to 1.5% by weight of the composition.

Preferably, the mixture of stabilising agents is present in an amount from 0.1 to 1.4%, from 0.2 to 1.2%, from 0.25 to 1.0%.

Advantageously, the mixture of stabilising agents is present in an amount from 0.3 to 0.8%, from 0.3 to 0.7%, from 0.3 to 0.6% and more advantageously from 0.3 to 0.5%.

The mixture of stabilising agents is typically present in an amount of 0.4%.

These ranges of stabilising agents have the advantage of stabilizing the composition according to the invention for at least one month in usual storage conditions, i.e. at a temperature comprised from 4°C to 10°C, without thickening or jellifying the composition at a point that it would no longer be easily drinkable.

Preferably, the composition according to the invention further comprises a mineral salt, such as for example potassium chloride, tripotassium citrate, potassium carbonate, dimagnesium phosphate, magnesium chloride, sodium chloride, calcium carbonate, betaine.

Preferably, the mineral salt is a carbonate salt, such as CaCO₃.

Preferably, the mineral salt is a calcium salt, such as CaCO₃.

Without willing to be bound by theory, mineral salts act as stabilizing agents by helping the prevention of intra- and intermolecular hydrogen bonds formation in the β-glucan polymer molecules, and thus prevent the formation of unpleasant aggregates.

When the mixture of stabilising agents comprises at least a pectin, the composition preferably further comprises a calcium salt. Without willing to be bound by theory, this calcium salt is believed to help the structuring of pectin-base gel.

Preferably, the mineral salt is present in an amount from 0.001% to 1% by weight of the composition, preferably from 0.005% to 0.5%, advantageously from 0.01 to 0.1%.

The mixture of stabilising agents of the invention is for example a mixture of two hydrocolloids, a mixture of three hydrocolloids, a mixture of one hydrocolloid and a mineral salt, a mixture of two hydrocolloids and a mineral salt, a mixture of three hydrocolloids and a mineral salt.

The composition according to the present invention is typically meant to reduce the cholesterol level and to control the glycemic index.

Accordingly, it is preferred that the composition according to the present invention contains less than 11 %, preferably less than 7%, advantageously less than 5%, less than 1% and more advantageously less than 0.5% of starch by weight of the composition.

Most preferably, the composition according to the present invention contains no starch.

Preferably, the composition of the present invention is a reduced-calorie, light or even low-calorie product.

Accordingly, the composition of the present invention preferably contains from 0% to 14% of sugars by weight of the composition, more preferably less than 10%, advantageously less than 5%, more advantageously less than 2%.

In the framework of the present application, the term "sugars" refers to monosaccharides, such as glucose, fructose and galactose, and to disaccharides, such as sucrose, maltose and lactose, and to any saccharide-type compound that provides a significant caloric content in typical usage amounts.

It is preferred that the composition according to the present invention contains less than 5%, preferably less than 2%, advantageously less than 1%, less than 0.5% and more advantageously less than 0.1 % of alcohol by weight of the composition.

Preferably, the composition of the present invention is an alcohol-free product.

Preferably, the composition according to the invention further comprises a flavouring agent, or a mixture of flavouring agents, preferably used in the composition in any suitable amount or concentration effective to achieve the level of taste desired.

The flavouring agent is preferably present in an amount from 0% to 3% by weight of the composition, more preferably less than 2%, advantageously less than 1%.

Flavouring agents include fruit flavours, plant flavours, spice flavours, flower flavours, among others.

As used herein, the term "fruit flavour" refers to any fruit fraction, fruit component (e.g., rind, zest, pith, pericarp, pulp, leaf, stem, seed, and the like), from the named fruit (FTNF) flavour (e.g., a combination of fruit essence, fruit oil and/or fruit flavour, such as, e.g., an orange from the named fruit flavour), fruit extract (e.g., expressed, absorbed, macerated, distilled and the like), fruit oil (e.g., essential oil, folded essential oil), fruit essence, fruit puree, fruit aroma and the like that can be added to a food product to enhance flavour (e.g., to provide and/or enhance one or more high note flavours).

In certain exemplary embodiments, one or more citrus fruit flavours are used. The citrus flavour may include one or more of an orange fraction, an orange component, an orange extract, an orange essential oil, an orange folded essential oil, an orange aroma, and an orange essence. The citrus flavour may also include one or more of a fraction, component, extract, essential oil, folded essential oil, aroma, or essence of grapefruit, lemon, lime, or tangerine, among others. The citrus flavour may also include chemical compounds extracted from natural sources or synthetically produced e.g., limonene, octanol and its derivatives, acetaldehyde, α-pinene, β-pinene, sabinene, myrcene, octanal, linalool, carene, decanal, citral, sinensal, among others.

As used here, the term "plant flavour" refers to flavours derived from parts of a plant other than the fruit. As such, plant flavours can include those flavours derived from essential oils and extracts of nuts, bark, roots and leaves. Examples of such flavours include cola flavours, tea flavours, spice flavours and the like, and mixtures thereof. Non-limiting examples of spice flavours include anise, cassia, clove, cinnamon, pepper, ginger, vanilla, cardamom, coriander, root beer, sassafras, ginseng, and others. Flavouring agents can be in the form of an extract, oleoresin, juice concentrate, bottler's base, or other forms known in the art.

As used herein, the term "flower flavour" refers to any flower fraction (e.g., petals) or flower extract (macerated or expressed).

Preferred flavouring agents are for example selected from the group consisting of fruit, vegetable or plant aromas, and may be, but are not limited to, lemon, lime, orange, pear, peach, apricot, mango, pineapple, banana, grapefruit, strawberry, raspberry, blackcurrant, cherry, passion fruit, water melon, papaya, cranberry, currant, apple, vanilla, chocolate, coffee, mint, tomato, cucumber, carrot, or mixture thereof.

Preferably, the composition according to the invention further comprises a fruit or vegetable juice concentrates, preferably used in the composition in any suitable amount or concentration effective to achieve the level of taste desired.

The fruit or vegetable juice concentrate is preferably present in an amount from 0% to 30% by weight of the composition, more preferably from 0 to 20%, advantageously from 0% to 15%, more advantageously from 1% to 10%, said juice concentrates being preferably selected from the group consisting of fruit or vegetable concentrates may be, but is not limited to, lemon, lime, orange, pear, peach, apricot, mango, pineapple, banana, grapefruit, strawberry, raspberry, blackcurrant, currant, cherry, passion fruit, water melon, papaya, cranberry, apple, tomato, cucumber, carrot, or mixture thereof.

Preferably, the composition according to the invention further comprises an aqueous flower extract, suitable for food composition, obtained by maceration of infusion or fresh or dry flowers into water. Conveniently, said aqueous flower extract, such as hibiscus extract, is used to prepare the composition of the invention.

In certain embodiment, the composition may include a vegetable component, including, e.g, but not limited to, one or more vegetable juices, extracts, powders, skins, rinds, grinds, roots, pulps, homogenised pulps, purees or any combination thereof. The vegetable component can be used in the composition in any suitable amount or concentration effective to achieve the level of taste or texture desired.

Preferably, the composition according to the invention further comprises at least one sweetener, preferably present in an amount or concentration effective to achieve the level of sweetness desired.

The term "sweetener", also called "sugar substitute", is a food additive, natural or synthetic, that duplicates the effect of sugar in taste, usually with less food energy. The sensation of sweetness caused by these compounds is calibrated on the sweetness of sugar (also called sucrose). Techniques to determine such sweetness are well-known from the skilled person. The sweetness of a sweetener is for example determined according to the following procedure.

Samples of water that have been artificially sweetened to varying degrees are presented to tasters of a taste panel. First, the tasters are given plain water, and then, they drink samples with higher and higher concentrations until they start to taste something different (not necessarily sweet). When half the test population can detect a change in the water, the "threshold value" for the sweetener is reached. The relative sweetness is then measured by comparing the threshold value of the sweetener with the one of sugar.

Preferably, the sweetener is at least 30 times as sweet as sugar.

Preferably, the sweetener is a natural or artificial non-nutritive sweetener, i.e. a sweetener which does not provide significant caloric content in typical usage amounts.

The sweetener is preferably present in an amount from 0% to 0.05% by weight of the composition.

The sweetener is preferably selected from the group consisting of rebaudioside A, steviol glycosides, *Stevia rebaudiana* extract, Lo Han Guo, mogroside V, monatine, glycyrrhizin, thaumatin, monellin, brazzein, cyclamate, acesulfame K, sucralose, aspartame, saccharine, neohesperidin dihydrochalcone, neotame, or mixture thereof.

More preferably, the sweetener is preferably selected from the group consisting of steviol glycosides and *Stevia rebaudiana* extract.

In certain embodiment, a combination of one or more sweeteners is used to provide the sweetness and other aspects of desired taste profile and nutritive characteristics.

Preferably, the composition according to the invention further comprises an acidifying agent, preferably selected from the group consisting of organic acids such as citric acid, malic acid, lactic acid, tartaric acid, adipic acid, gluconic acid, fumaric acid, succinic acid, maleic acid, orthophosphoric acid, or mixture thereof.

More preferably, the composition according to the invention further comprises an acidifying agent selected from the group consisting of citric acid and lactic acid, or mixture thereof.

The acidifying agent is preferably a mixture of lactic acid and citric acid.

The acidifying agent is preferably present in an amount from 0% to 1% by weight of the composition.

As known from the skilled person, acids chosen and the amount used will depend, in part, on the other ingredients, the desired shelf life of the composition, as well as effect on the composition pH, titratable acidity, and taste. Organic acids used in certain exemplary embodiments of the composition disclosed here can serve one or more additional functions, including, for example, lending tartness to the taste of the composition, enhancing palatability, increasing thirst quenching effect, and acting as a mild preservative.

The composition according to the invention may further comprise at least one ingredient selected from the group consisting of a taste modifier, a vitamin, a mineral, a buffering agent, a colorant and a preservative.

Taste modifiers may provide their own characteristic flavour, or may have little or no flavour impact by themselves. Taste modifiers have any one or more of the properties of reducing, masking, or eliminating undesirable taste characteristics, or enhancing desirable taste characteristics, for example, by controlling one or more of sweetness, sourness, bitterness, saltiness, mouthfeel, or taste temporal effects. Non-limiting examples of undesirable taste characteristics reduced by taste modifiers include one or more of bitter aftertaste, metallic aftertaste, astringency, thin mouthfeel, harshness, delayed sweetness onset, lingering sweetness, excess sourness, and other off-notes. Non-limiting examples of desirable taste characteristics enhanced by taste modifiers include one or more of sweetness intensity or impact, fullness or body, and smoothness, among others. Non-limiting examples of taste modifiers include organic acids (e.g., citric acid, malic acid, tartaric acid, lactic acid, adipic acid, fumaric acid, gluconic acid, succinic acid, maleic acid, among others), propylene glycol, glycerol, ethanol, and commercially available products (e.g., Symrise™ Natural Flavor, Sweetness Enhancer Type SWL 196650, Firmenich Natural Flavor (Modulasense™ Type) 560249 T, and Firmenich™ Natural Flavor (Modularome™ Type) 539612 T, among others). It will be within the ability of those skilled in the art, given the benefit of this disclosure, to select suitable additional or alternative taste modifiers for use in various embodiments of the composition disclosed here.

Certain embodiments of the composition disclosed here may contain one or more added vitamins, e.g., added Vitamin A (including Vitamin A precursors such as beta carotene), Vitamin B₁ (i.e., thiamine), Vitamin B₂ (i.e., riboflavin), Vitamin B₃ (i.e., niacin), Vitamin B₆, Vitamin B₇ (i.e., biotin), Vitamin B₉ (i.e., folic acid), Vitamin B₁₂ (i.e., cobalamin), Vitamin D, and Vitamin E (i.e., tocopherols and tocotrienols), and Vitamin K, and combinations thereof.

It is preferred that the composition according to the present invention contains no Vitamin C (i.e. ascorbic acid).

Certain embodiments of the composition disclosed here may contain one or more added minerals, e.g., added calcium, potassium, magnesium, phosphorous, zinc, and iron, among others.

Certain embodiments of the composition disclosed here also may contain small amounts of buffering agents to adjust pH. Such pH adjusters include, e.g., the sodium and potassium salts of citric, tartaric, and lactic acids. The amount included will depend, of course, on the type of buffering agents and on the degree to which the pH is to be adjusted.

Colorant, or color additive, may be any dye, pigment or substance that imparts color when it is added to food or drink. Colorants may be in forms consisting of liquids, powders, gels and pastes. The skilled person is able to select among additives E100-E199 the food colorant or mixture of food colorants suitable to obtain the desired color.

Common food colorants include for example caramel coloring (E150), annatto (E160b), chlorophyllin (E140), cochineal (E120), betanin (E162), turmeric (curcuminoids, E100), saffron (carotenoids, E160a), paprika (E160c), lycopene (E160d), elderberry juice, pandan and butterfly pea.

Preservative is a naturally occurring or synthetically produced substance that is added to products such as foods to prevent decomposition by microbial growth (bacteria or fungi, including mold) or by undesirable chemical changes.

The skilled person is able to select among additives E200-E299 the food preservative or mixture of food preservatives suitable to obtain the desired effect.

Common antimicrobial preservatives include for example sorbic acid and its salts, benzoic acid and its salts, calcium propionate, sodium nitrite, sulfites (sulfur dioxide, sodium bisulfite, potassium hydrogen sulfite, etc.) and disodium EDTA (EthyleneDiamineTetraacetic Acid). Antioxidant food preservatives include BHA (Butylated HydroxyAnisole), BHT (Butylated HydroxyToluene), TBHQ (Tertiary ButylHydroQuinone) and propyl gallate. Preservatives also include natural antioxidants. Natural antioxidants include for example plants extract, such as rosemary extract.

The composition according to the invention typically has a dynamic viscosity comprised from 5 mPa.s and 1500 mPa.s.

Preferably, the composition has a dynamic viscosity from 10 mPa.s and 1 000 mPa.s, more preferably less than 500 mPa.s, advantageously less than 300 mPa.s, more advantageously less than 150 mPa.s.

These ranges of viscosity are suitable for convenient drinkability of the composition of the invention, at a temperature from 4°C to 80°C

Dynamic viscosity is defined as the tangential force per unit area required to move one horizontal plane with respect to the other at unit velocity when maintained a unit distance apart by the fluid.

The dynamic viscosity is typically measured according to a method well-known by the skilled person, using a vibro-viscometer (AnD SV-10) equipped with vibrating blades immerged in the sample under analysis, the temperature being controlled by a thermostatic bath. The resistance of the sample to the oscillating blades is correlated to the sample viscosity. Thus, by measuring said resistance, the viscosity of the sample is determined, according to the thermostatic temperature.

The composition according to the invention is for example a drinkable beverage, which is preferably consumable at a temperature comprised between 4°C and 80°C.

The composition of the invention is for example a flavoured soup or a flavoured cold drink, optionally sweetened with a non-nutritive sweetener.

More preferably, the composition according to the invention is consumable between 4°C and 25°C, and preferably between 4°C and 10°C.

Even at these relatively cold temperatures, the viscosity of the composition does not prevent its drinkability.

The dry matter of the composition according to the invention is typically comprised between 0.4% and 20%, preferably comprised between 1% and 10%, advantageously between 1.8% and 5.0%.

The pH of the composition according to the invention is typically comprised between 2.5 and 5.5, preferably between 2.6 and 5, more preferably between 2.8 and 4.5, advantageously between 3 and 4.

Preferably, the composition according to the invention further comprises an oil, preferably a vegetable or animal oil.

Said oil is preferably present in an amount from 0% to 2%, advantageously in an amount of 1 % by weight of the composition.

Said oil is preferably selected from the group consisting of fish oil rich in omega-3, arachid oil, avocado oil, safflower oil, colza oil, olive oil, sunflower oil, grapeseed oil, sesame oil, soybean oil, corn germ oil, wheat germ oil, flax oil and nut oil.

According to one embodiment, the composition according to the invention is in the form of an oil-in-water emulsion.

The oil phase typically consists in a flavouring agent such as an essential oil or a vegetable oil.

The oil phase preferably represents from 0 to 10% in weight of the composition, preferably from 0 to 5%, advantageously from 0 to 1%.

A preferred composition according to the invention comprises at least one hydrocolloid as described above and at least one mineral salt as described above.

Another preferred composition according to the invention comprises at least two hydrocolloids as described above, and optionally at least one mineral salt as described above.

Another preferred composition according to the invention comprises:
- from 1.0 to 2.0%, preferably from 1.0 to 1.5%, advantageously from 1.0 to 1.3%, more advantageously from 1.0 to 1.2%, for example 1.0% by weight of β-glucan polymer,
- from 0.1 to 0.5%, preferably from 0.1 to 0.3%, for example 0.2% by weight of a mixture of xanthan gum and arabic gum, generally in a 1:1 weight ratio, and
- from 0.1 to 0.5%, preferably from 0.1 to 0.3%, for example 0.2% by weight of a pectin.

Optionally, said composition further comprises from 0.005 to 0.05%, for example 0.01 % by weight of a carbonate salt, such as CaCO₃.

Said composition also preferably further comprises at least one acidifying agent, one flavouring agent, sugar, one colorant and/or one sweetener, preferably non-nutritive.

### Methods of preparation

The method of preparation of a composition as defined in the section "Compositions" above comprises a step of forming an aqueous solution, which provides a homogeneous and stable solution of β-glucan and stabilising agents in water, which preferably does not contain any aggregates of particles of β-glucan in suspension in the solution.

According to one embodiment, the step of forming the aqueous solution comprises:
- a step of addition into water of a β-glucan-based compound, the mixture of stabilising agents as defined in the section "Compositions" above, and optionally mineral salts, sugars, sweeteners as defined in the section "Compositions" above, at a dry powder state to obtain a mixture, and
- a step of mechanically stirring said mixture, at a temperature comprised between 80°C and 100°C, to obtain said aqueous solution.

The term "β-glucan-based compound" refers to a source of β-glucan, such as a purified extract from cereals such as oats or barley, highly concentrated in β-glucan polymer. Such purified extract may be commercially available.

The β-glucan-based compound preferably contains at least 50% by weight of β3-glucan polymer, advantageously at least 70%, typically around 75%, the remaining generally consisting in proteins, fibres and lipids contained in the cereals.

Glucagel™ (commercialised by DKSH) is an example of β-glucan-based compound suitable for the method of the invention. Glucagel™ comprises 75% of β-glucan polymer, 5% of fibres other than β-glucan, 4% of proteins, 3% of starch and 1.5% of lipids.

Conveniently, the β-glucan-based compound and the mixture of stabilising agents are added into hot water portion by portion.

Alternatively, it is also possible to add water on the β-glucan-based compound while stirring, said water being added portion by portion.

Alternatively, it is also possible to add a little amount of ethanol on the β-glucan-based compound (about 6 mL ethanol per gram of β-glucan polymer) to wet the powder before adding hot water.

Advantageously, the mixture is stirred at a temperature and for a period of time suitable to obtain a homogeneous and stable solution, which preferably does not contain any particles of β-glucan in suspension in the solution.

Typically, the mixture is stirred at a temperature comprised between 80°C and 100°C for a period comprised from 1 min to 2 h, preferably from 5 min to 1 h.

The step of mechanically stirring is for example carried out with a mixing device, preferably a high-speed disperser (above 1000 rpm), such as a rotor-stator mixer (such as an Ultra-turrax, a VMI or a Silverson mixer) or a deflocculation mixer (such as a VMI), or any apparatus suitable to homogenize the mixture.

Conveniently, a combination of different kinds of mixer can be used, such as a deflocculation mixer then a rotor-stator mixer.

The stirring speed is preferably comprised between 100 rpm and 16 000 rpm, preferably from 1000 rpm to 16 000 rpm.

Advantageously, in particular when the β-glucan-based compound comprises proteins, the step of mechanically stirring is followed by a step of boiling the aqueous solution, preferably at a temperature of 100°C, advantageously for 30 min to 120 min, and a step of centrifugation and separation of the solids.

The step of boiling aims at flocculating the proteins eventually comprised in the aqueous solution obtained after the step of mechanically stirring. The flocculated proteins are conveniently eliminated via the step of centrifugation.

According to this embodiment, the other ingredients, such as flavouring agents, fruit or vegetable juices concentrates, sweeteners, acidifying agents, and the above-mentioned additives, are further added to obtain the composition of the invention, before its conditioning.

Preferably, the method of the invention further comprises a step of thermal treatment of the composition before or during the conditioning of said composition.

Alternatively, the method of the invention further preferably comprises a step of thermal treatment of the composition after the conditioning of said composition.

Such thermal treatment aims at preventing the microbial growth in the composition during its storage period.

For example, the composition is conditioned at hot temperature, generally comprised from 80 to 100°C, or the composition is treated by any type of pasteurisation.

According to one embodiment, the composition is treated by pasteurisation before its conditioning, for example by flash-pasteurisation.

According to one embodiment, the composition is treated by pasteurisation after its conditioning, for example by tunnel-pasteurisation.

### Beverage unit

The beverage unit according to the invention preferably enables the administration of at least 3 g of β-glucan polymer during the same day, particularly during a 12 h period.

Advantageously, the beverage unit is a beverage of 200 mL to 500 mL, preferably of 200 m L to 330 mL.

According to one embodiment, the beverage unit is a beverage of 330 mL.

For example, a beverage unit of 330 mL of a composition according to the invention comprising 1.0% by weight of β-glucan enables the administration of 3.3 g of β-glucan, and is easily drinkable, even in the framework of a daily diet.

According to another embodiment, the beverage unit is a beverage of 200 mL.

For example, a beverage unit of 200 mL of a composition according to the invention comprising 1.5% by weight of β-glucan enables the administration of 3.0 g of β-glucan, and is easily drinkable, even in the framework of a daily diet.

### Methods of prevention of diseases

As known from the skilled person, β-glucan polymers, when used at sufficient levels, have beneficial effects in the reduction of the cholesterol level and of the glycemic index, thereby decreasing the risk of onset of cardiovascular diseases and of the metabolic syndrome.

The present invention therefore relates to a composition, as defined above, for use for the prevention of a cardiovascular disease in a subject.

The present invention also concerns the use of a composition, as defined above, for the manufacture of a functional food intended to the prevention of a cardiovascular disease.

The present invention also concerns a method for preventing a cardiovascular disease in a subject, comprising administering to a subject in need thereof a prophylactically effective amount of a composition, as defined above.

In the context of the invention, the term "cardiovascular disease" refers to a disease that involves the heart or blood vessels (arteries and veins). More particularly, a cardiovascular disease according to the invention denotes a disease, lesion or symptom associated with an atherogenesis process that affects the cardiovascular system. It includes especially the conditions in which an atheroma plaque develops as well as the complications due to the formation of an atheroma plaque (stenosis, ischemia) and/or due to its evolution toward an acute ischemic stroke (thrombosis, embolism, infarction, arterial rupture).

Cardiovascular diseases include coronary artery disease, coronary heart disease, hypertension, atherosclerosis, in particular iliac or femoral atherosclerosis, angina pectoris, thrombosis, heart failure, stroke, vascular aneurysm, vascular calcification, myocardial infarction, vascular stenosis and infarction, and vascular dementia. Preferably, the cardiovascular disease according to the invention is selected from the group consisting in coronary artery disease, hypertension, atherosclerosis, vascular aneurysm, vascular calcification, vascular dementia and heart failure.

The present invention is also drawn to a composition, as defined above, for use for the prevention of a metabolic syndrome in a subject.

The present invention also concerns the use of a composition, as defined above, for the manufacture of functional food intended for the prevention of a metabolic syndrome.

The present invention also relates to a method for preventing a metabolic syndrome in a subject, comprising administering to a subject in need thereof a prophylactically effective amount of a composition, as defined above.

In the context of the invention, the term "metabolic syndrome" refers to a multiplex risk factor for cardiovascular disease comprising the 6 following components: abdominal obesity, atherogenic dyslipidemia, raised blood pressure, insulin resistance with or without glucose intolerance, proinflammatory state and prothrombotic state. The metabolic syndrome is more specifically defined in Grundy et al. (2004) Circulation 109:433-438.

As used herein, the term "functional food" refers to a natural or processed food that contains known biologically-active compounds which when in defined quantitative and qualitative amounts provides a clinically proven and documented health benefit.

As used herein, the term "preventing" or "prevention" means decreasing or cancelling the risk of appearance of the disease concerned.

Compositions of the invention will be administered to a subject in an amount sufficient to delay, reduce, or prevent the onset of clinical or subclinical disease. An amount adequate to accomplish this purpose is defined as a "prophylactically effective amount". Determination of an appropriate dosage amount and regimen can readily be determined by those skilled in the art. Amounts effective for this use may depend on the severity of the disease or condition and the weight and general state of the patient, but are typically of at least 3 g of β-glucan polymer per day per subject. The total effective amount of β-glucan polymers present in the compositions of the invention can be administered to a mammal as a single dose, or can be administered using a fractionated treatment protocol, in which multiple doses are administered over a more prolonged period of time.

The prophylactically effective amount of the compositions of the invention and used in the methods of this invention applied to mammals (e.g., humans) can be determined by those of skill in the art with consideration of individual differences in age, weight and the condition of the mammal. The agents of the invention are administered to a subject (e.g. a mammal, such as human, mouse, livestock (e.g., cattle, sheep, or pigs), domestic pet (e.g., cat or dog)) in an effective amount, which is an amount that produces a desirable result in a treated subject. Such prophylactically effective amounts can be determined empirically by those of skill in the art.

The compositions of the invention are preferably used for maintaining a normal blood cholesterol concentration in a subject.

As used herein, the term "maintaining" means preventing significant variations in a level of interest around a given value.

As used herein, the term "normal blood cholesterol concentration" refers to the range of blood cholesterol concentrations or the mean blood cholesterol concentrations measured in a healthy subject. Typically, a normal blood cholesterol concentration is below 200 mg/dL or 5.2 mmol/L.

The compositions of the invention are also preferably used for decreasing glycemic index of a food or a meal ingested by a subject.

As used herein, the term "glycemic index" or "GI" refers to a classification criterion of food comprising carbohydrates, based on their effects on glycemia during the two hours following their ingestion. The glycemic index of a food is given relative to a reference food to which the index 100 is given (typically glucose or white bread).

The compositions of the invention are particularly useful for decreasing glycemic index during a meal, in particular when administered, preferably ingested, simultaneously with the meal.

In the context of the invention, a "subject" denotes a human or non-human mammal, such as a rodent (rat, mouse, rabbit), a primate (chimpanzee), a feline (cat), or a canine (dog). Preferably, the subject is human. The subject according to the invention may be in particular a male or a female. The subject according to the invention is preferably an adult.

In a particular embodiment, the subject to be treated has a normal or mildly elevated blood cholesterol concentration.

In another particular embodiment, the subject to be treated is obese.

As used herein, the term "obesity" or "obese" refers to a medical condition in which excess body fat has accumulated to the extent that it may have an adverse effect on health, leading to reduced life expectancy and/or increased health problems. Obesity is typically determined by assessing the body mass index (BMI), a measurement which compares weight and height. In particular, people are defined as overweight if their BMI is between 25 kg/m² and 30 kg/m², and obese when it is greater than 30 kg/m².

The compositions of the invention are preferably administered by the oral route. They are typically ingested, preferably before or during a meal.

### Examples

### Example 1

This example demonstrates that a mixture of stabilizing agents is able to stabilize an aqueous composition of β-glucan polymer.

### Ingredients

- Glucagel™, commercialised by DKSH,
- Fibregum™ (gum Arabic), commercialised by Nexira,
- Thixogum S™ (gum arabic/gum xanthan 1:1), commercialised by Nexira,
- Rheogel® (gum xanthan), commercialised by Nexira,
- Guar gum (Meyprodor 50), commercialised by Danisco,
- Potassium citrate (Tripotassium citrate), commercialised by Glanbia Nutritionals,
- Grindsted Pectin RS 461 S1 and Pectin RS 450, commercialised by Danisco,
- Pectin AE and Pectin AL, commercialised by Naturex,
- Calcium carbonate, commercialised by Chimie-Plus Laboratories, and
- Citric acid anhydrous, commercialised by DSM.

### Preparation of test compositions

Test compositions comprising β-glucan polymer were prepared using commercially available Glucagel™ as source of β-glucan polymer.

Glucagel™ comprises, by weight:
- 75% of β-glucan polymer,
- 5% of fibres other than β-glucan,
- 4% of proteins,
- 3% of starch, and
- 1.5% of lipids.

The method of preparation of the test compositions was as follows. Glucagel™ is mixed together with the stabilising agents and the optional salts, in order to obtain a mixture of powders. This mixture is then added to a predetermined amount of water to obtain a suspension. The amount of water is calculated according to the concentration in β-glucan polymer desired in the final test composition. The resulting suspension is then mechanically stirred for 1 to 3 min and heated to 80°C, in order to obtain a solution of β-glucan polymer.

At the end of the stirring, citric acid (50% w/w) is added to the hot solution to adjust the pH to 3.5 and the resulting solution is conditioned in a bottle, before it has cooled down, and is let to rest at a storage temperature of 4°C.

The stability period of the different test compositions was then measured and presented in **Table 1.** The stability period of a test composition is the period during which said test composition remains stable, without formation of aggregates, sedimentation or phase separation phenomenon, and without any change of aspect that would affect its drinkability.

**Table 1: Stability period**

| Example | Stirring method | Ingredients | Stability period |
|---|---|---|---|
| 1 | Deflocculation mixer (1500 rpm) | 1.44% Glucagel | 3 days at 4°C |
| 2 | Deflocculation mixer (1500 rpm) | 0.58% Glucagel | 3 days at 4°C |
| 3 | Magnetic stirrer (100 to 800 rpm) | 1.6% Glucagel 0.5% Fibregum | 2 days at 4°C |
| 4 | Magnetic stirrer (500 rpm) | 1.44% Glucagel 0.5% Guar gum | 2 days at 4°C |
| 5 | Magnetic stirrer (500 rpm) | 1.44% Glucagel 1.0% Guar gum | 2 days at 4°C |
| 6 | Magnetic stirrer (500 rpm) | 1.44% Glucagel 0.25% Thixogum S 1.0% potassium citrate | > 1 month at 4°C |
| 7 | Deflocculation mixer (2000 rpm) + Rotor stator stirrer (15000 rpm) | 0.58% Glucagel 0.2% Thixogum S 0.2% pectin RS 461 0.01% CaCO₃ | > 1 month at 4°C |
| 8 | Deflocculation mixer (2000 rpm) + Rotor stator stirrer (13000 rpm) | 1.44% Glucagel 0.1% Thixogum S 0.1 % pectin RS 461 0.005% CaCO₃ | > 23 days at 4°C |
| 9 | Rotor stator stirrer (6500 rpm) | 1.44% Glucagel 0.2% Thixogum S 0.2% pectin RS 461 0.01% CaCO₃ | **> 6 months at 4°C** |
| 10 | Magnetic stirrer (500 rpm) | 1.44% Glucagel 0.2% Thixogum S 0.02% CaCO₃ | **> 6 months at 4°C** 15 days at 25°C |

Test compositions 1 and 2 show that a solution comprising the β-glucan base compound alone, without any stabilizing agent, is not stable more than 3 days at 4°C.

Test compositions 3 to 5 show that a stabilising agent alone (gum Arabic or guar gum) is not able to stabilize a solution of β-glucan for more than 2 days.

The above results show that a mixture of stabilising agents is thus necessary to stabilize the solution of β-glucan composition for at least 23 days at 4°C.

Test compositions 6 to 10 show that a mixture of at least two stabilising agents extends the stability of the composition at 4°C toat least 23 days (and even to six months for compositions 9 and 10), without affecting the drinkability of the composition.

### Preparation of a beverage

A drinkable beverage was prepared according to the ratios of test composition 9.

Glucagel (1.44%), Thixogum S (0.2%), pectin RS 461 (0.2%), and CaCO₃ (0.01%) were mixed with *Stevia* (0.01%) and sugar (3.3%). The resulting powder mixture was solubilised in hot water according to the procedure described above, using a deflocculation mixer (2000 rpm) and a rotor-stator mixer (15000 rpm). The hot resulting solution was then acidified to pH = 3.5 by addition of citric acid (50% w/w). A multi-fruit berry juice concentrate (3.3%, Döhler) was then added.

Compared to the test composition 9, the addition of these further ingredients did not affect the stability of the beverage, which exhibited the same properties of stability than the test composition.

### Example 2

This example demonstrates the beneficial effect of the composition according to the invention on glycemia in rats.

### Materials and methods

### Materials

Glucagel™, commercialised by DKSH
Thixogum S™ (gum arabic/gum xanthan 1:1), commercialised by Nexira
Grindsted Pectin RS 461 S1, commercialised by Danisco.
β -glucan polymer having a molecular weight of 160 kDa, commercialized by Megazyme
β -glucan polymer having a molecular weight of 270 kDa, commercialized by Megazyme
β -glucan polymer having a molecular weight of 590 kDa, commercialized by Megazyme

### Methods

A beverage comprising 7.5% starch represented the "meal" was eaten at the same time as the beverages to be studied.

Five beverages were tested:
1) a control beverage comprising only water,
2) a beverage comprising water, 1% β-glucan polymer having a molecular weight of 590 kDa (Megazyme), 0.2% of Thixogum S™, 0.2% pectin and 0.01% CaCO₃
3) a beverage comprising water, 1% β-glucan polymer having a molecular weight of 250 kDa (Glucagel), 0.2% of Thixogum S™, 0.2% pectin and 0.01% CaCO₃.

Ten rats were used for each category.

The rats were crammed with the beverages to be tested then with the beverage comprising starch. 13.3 mL of each beverage was used per kg of rat.

Blood sampling was carried out 0, 15, 30, 45, 60, 90 and 120 minutes after the meal.

Glycemia was analyzed using a glucometer (UltraTouch 2, Lifescan).

### Results

The area under the curve (AUC) of glycemia was determined for each category 120 minutes after the meal. **Table 2** shows the results obtained.

**Table 2: AUC of glycemia**

| **composition** | **AUC120** | **Decrease relative to control with water (1)** |
|---|---|---|
| 1 | 7771 | 0.0% |
| 4 | 6833 | -13.7%* |
| 5 | 7034 | -10.5%* |

| | | |
|---|---|---|
| * (p<0.04) | | |

These results show that the compositions according to the invention can induce a significant decrease in glycemia (p<0.04), said decrease being more important when the molecular weight of the β-glucan compounds administered is higher.

### Example 3

This example demonstrates the beneficial effect of the composition according to the invention on glycemia in rats. The beverage is compared to a solution of glucose with the same dry matter.

### Materials and methods

### Materials

Glucagel™, commercialised by DKSH
Thixogum S™ (gum arabic/gum xanthan 1:1), commercialised by Nexira
Grindsted Pectin RS 461 S1, commercialised by Danisco.
Glucose

### Methods

A beverage comprising 7.5% starch represented the "meal" was eaten at the same time as the beverages to be studied.

Six beverages were tested:
1) a control beverage comprising only water,
2) a beverage comprising water and 1,8% glucose,
3) a beverage comprising water, 1% β-glucan polymer having a molecular weight of 250 kDa (Glucagel™), 0.2% of Thixogum S™, 0.2% pectin and 0.01 % CaCO₃,
4) a beverage comprising water, 1% β-glucan polymer having a molecular weight of 250 kDa (Glucagel™).

Ten rats were used for each category.

The rats were crammed with the beverages to be tested then with the beverage comprising starch. 13.3 mL of each beverage was used per kg of rat.

Blood sampling was carried out 0, 15, 30, 45, 60, 90 and 120 minutes after the meal.

Glycemia was analyzed using a glucometer (UltraTouch 2, Lifescan) and insulinemia was assayed using an ELISA kit (kit Mercodia).

### Results

The area under the curve (AUC) of glycemia was determined for each category 120 minutes after the meal. **Table 3** shows the results obtained.

**Table 3: AUC of glycemia**

| **composition** | **AUC120** | **Decrease relative to control with water (1)** | **Decrease relative to control with water and glucose (2)** |
|---|---|---|---|
| 1 | 6800 | 0.0% | -4.99% |
| 2 | 7157 | +5.2% | 0.0% |
| 3 | 6441 | -5.28% | -10.01%* |
| 4 | 6383 | -6.13% | -10.81%* |

| | | | |
|---|---|---|---|
| * (p<0.03) | | | |

These results show that the compositions 3 and 4 according to the invention induce a significant decrease in glycemia compared to the composition 2 with a dry matter equivalent composed of glucose.

## Claims

1. An aqueous food composition, comprising at least one β-glucan polymer and at least one mixture of stabilising agents, wherein the β-glucan polymer is present in an amount from 0.4% to 3% by weight of the composition.

2. The composition according to claim 1, wherein the β-glucan polymer is present in an amount from 0.9% to 1.5% by weight of the composition.

3. The composition according to claim 1 or 2, wherein the average molecular weight of the β-glucan polymer is from 70 kDa to 2 000 kDa.

4. The composition according to any one of claims 1 to 3, wherein the mixture of stabilising agents comprises at least a hydrocolloid.

5. The composition according to any one of claims 1 to 4, wherein the mixture of stabilising agents comprises at least a hydrocolloid selected from the group consisting of arabic gum, xanthan gum and pectin.

6. The composition according to any one of claims 1 to 5, wherein the mixture of stabilising agents is present in an amount from 0.05% to 1.5% by weight of the composition.

7. The composition according to any one of claims 1 to 6, having a dynamic viscosity comprised between 5 mPa.s and 1500 mPa.s.

8. The composition according to any one of claims 1 to 7, wherein the composition is a drinkable beverage.

9. A method for preparing the composition according to any one of claims 1 to 8, comprising a step of forming an aqueous solution comprising at least one β-glucan polymer and at least a mixture of stabilising agents.

10. The method according to claim 9, wherein the step of forming the aqueous solution comprises:
- a step of addition into water of a β-glucan-based compound, the mixture of stabilising agents, and optionally mineral salts, sugars, sweeteners, at a dry powder state to obtain a mixture, and
- a step of mechanically stirring said mixture, at a temperature comprised between 80°C and 100°C, to obtain said aqueous solution.

11. A beverage unit comprising an amount of composition according to any one of claims 1 to 8 such that said beverage unit comprises an amount of β-glucan compound of 3 g or more.

12. Composition as defined in any one of claims 1 to 8, for use for the prevention of a cardiovascular disease or metabolic syndrome in a subject.

13. The composition as defined in any one of claims 1 to 8, for use for maintaining a normal blood cholesterol concentration in a subject.

14. The composition as defined in any one of claims 1 to 8, for use for decreasing glycemic index of a food or a meal ingested by a subject.
